# EUROPEAN PATENT SPECIFICATION

(11) **EP 2 644 207 B1**
(45) Date of publication and mention of the grant of the patent: **19.11.2014**
(21) Application number: 13161350.7
(22) Date of filing: 27.03.2013
(51) Int. Cl.: A61L 2/20, A61L 9/015

(54) **Device for gas disinfection**
Vorrichtung zur Gasdesinfektion
Appareil pour stérilisation de gaz

(30) Priority: 27.03.2012 PL 12089212 U
(43) Date of publication of application: 02.10.2013
(73) Proprietor: Adamowy.pl Adam Chojnacki, 02-796 Warszawa (PL)
(72) Inventor: Chojnacki, Adam, 02-796 Warszawa (PL)
(74) Representative: Karcz, Katarzyna

(56) References cited:
- JP-A- H01 224 893
- JP-A- H11 272 946
- JP-A- 2004 152 007

## Description

The present invention relates to an apparatus for gas disinfection, in particular for gas disinfection of vehicle interiors, the apparatus being designed for self-service. The apparatus may be used for example at petrol stations, vehicle service centres, car washes, parking lots. The present solution has a novel structure, in which the apparatus for gas disinfection is combined with a fare collection unit.

Gas disinfection is used to purify the air in rooms and vehicles and to remove odours therefrom caused e.g. by smoking, the presence of animals, chemicals (e.g. paints, varnishes) as well as by flooding or burning.

The gas which may be used for disinfection is ozone, as well as ionized air i.e. the air that is enriched with negative ions or negative and positive ions. As a result of ozonation or ionized air operations, the disinfected interior is clean, fresh and free of viruses, bacteria and other health-hazardous compounds.

In the prior art, apparatuses for gas disinfection in the form of ozonation and ionization equipment are known.

U.S. patent specification US4857277 discloses an apparatus comprising a housing for a fan and an ozone generator, in which an air filter is located within the plane of the housing.

U.S. patent specification US3078526 discloses a footwear ozonation machine fitted with a coin collector. The machine has a housing for a shoe rack in the form of two profiled tubes on which ozonated shoes are placed. The ozonation machine known from the prior art is not appropriate for ozoning vehicles.

Japanese patent specification JP2004152007 discloses a compact ozone generating device capable of generating the optimal volume of ozone depending on the surrounding temperature. JP2004152007 also discloses a vending machine, for example, a vending machine that sells food and beverages, capable of effectively carrying out sterilization and disinfecting by using this device.

Japanese patent specification JPH01224893 discloses an ozone generator for in-machine sterilization of drink vending machine. A control system discharges low concentration ozone into the in-machine space and injects concentrate ozone into a water circuit.

Japanese patent specification JPH11272946 discloses a vending machine having deodorization function. The object of the invention is to easily and economically deodorize the waste liquid container and the inside of the vending machine by attaching a deodorizing device provided with an ozone processing part.

Each device disclosed in above-mentioned Japanese patent specifications uses ozone to disinfecting the inside of the vending machines. None of the machine is suitable for discharging ozone outside of the machine.

The apparatus for gas disinfection according to the invention, in particular for refreshing vehicles, comprises a housing and a unit for generating disinfecting gas positioned within the housing, and a fan for sucking in the air and for supplying the air to the unit for generating disinfecting gas, the fan being connected with the unit for generating disinfecting gas, the apparatus further comprising a supply pipe coupled with the unit for generating disinfecting gas, the supply pipe communicating with the outside of the apparatus, wherein the apparatus is equipped with a fare collection unit, coupled with a module for switching the unit for generating disinfecting gas.

Preferably, the apparatus comprises an air filter positioned within the plane of the housing.

Preferably, the fare collection unit is a unit for receiving coins or tokens.

Preferably, the fare collection unit is a unit for receiving banknotes.

Preferably, the fare collection unit is a unit for receiving payment cards.

Preferably, the fare collection unit is a unit for accepting payments by SMS.

Preferably, the unit for generating disinfecting gas is a unit for generating ozone.

Preferably, the unit for generating disinfecting gas is a unit for generating negative ions.

Preferably, the unit for generating disinfecting gas is a unit for generating negative and positive ions.

Preferably, the apparatus is equipped with a display.

Preferably, the apparatus is equipped with an element for stopping the process of ozonation.

Preferably, the apparatus is equipped with a handle for the pipe.

Preferably, the housing of the apparatus comprises an access door.

Preferably, the apparatus is equipped with a heater positioned within the housing.

Preferably, the apparatus is equipped with an element adjusting the amount of the dispensed gas.

The advantage of the invention is that due to its construction it may be used by any untrained user and due to the presence of the fare collection unit the apparatus is fully self-service.

Moreover, due to the fact that the apparatus for gas disinfection is equipped with the fare collection unit, the payment is done automatically at the place of its installation. Therefore, the apparatus may be used by anyone, without incurring additional costs which would include the salary of the personnel performing such services.

Additionally, the apparatus for gas disinfection in the form of a vending machine allows to popularise air ozoning and refreshing services in vehicles and provide access to those services to a much wider range of users.

An example of a preferred embodiment of the apparatus for gas disinfection of the invention will be shown in detail in the accompanying drawing in which:
Fig. 1 shows a general front view of the apparatus for gas disinfection of the invention;
Fig. 2 shows a perspective view of the apparatus for gas disinfection shown in Fig. 1;
Fig.3 shows the internal structure of the apparatus.

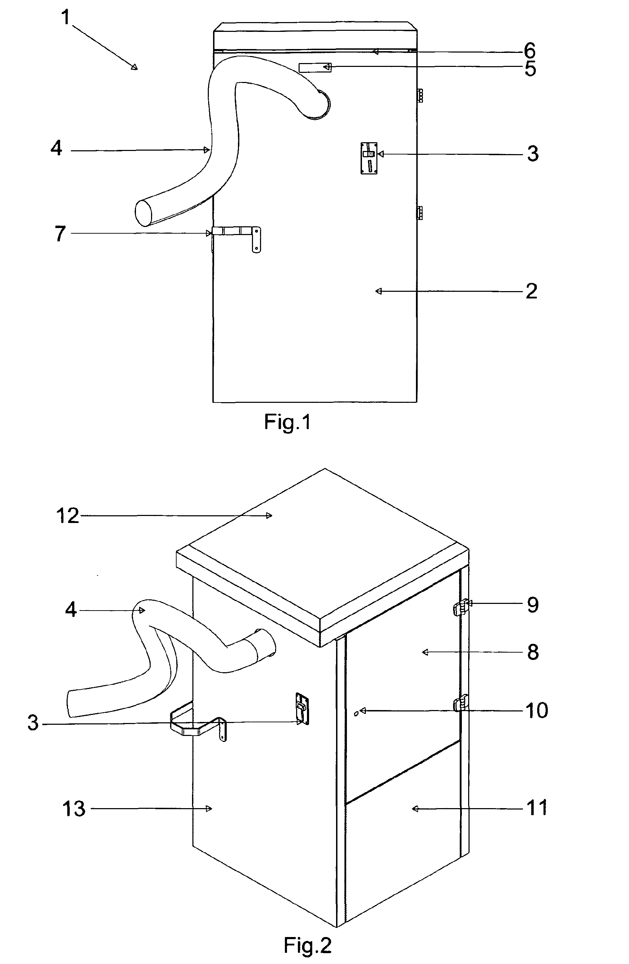

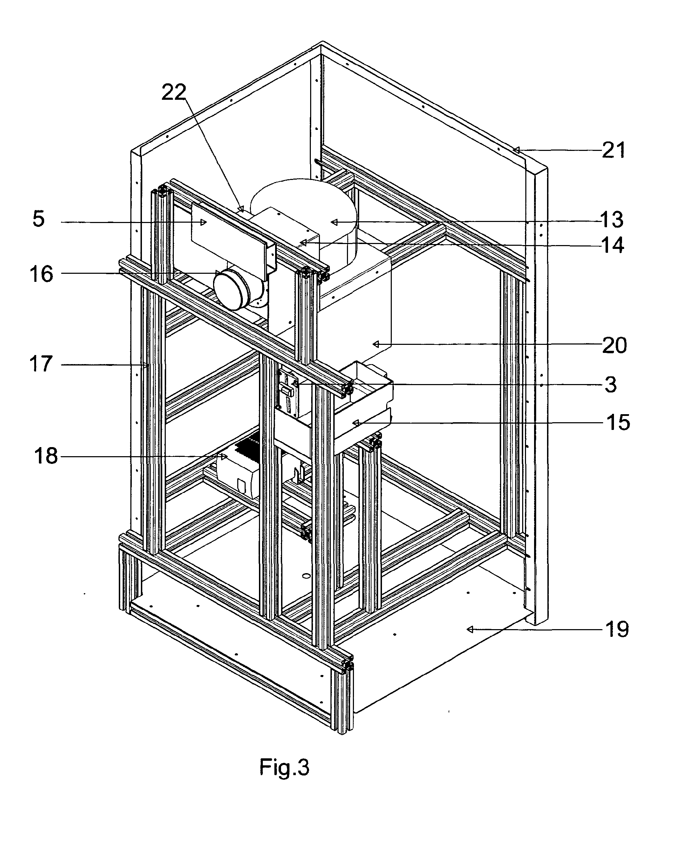

The gas disinfection apparatus 1 shown in Fig. 1 has a housing 2, an unit for generating disinfecting gas 14 having the form of a unit for generating ozone positioned within the housing (as shown in detail in Fig. 3), a supply pipe 4 and a fare collection unit 3. The fare collection unit 3 may be any unit collecting any means of payment known from the prior art, such as a coin collector, a unit for receiving notes, a unit for receiving cards or an unit accepting payments by SMS. The fare collection unit 3 is accessible from outside of the housing and is coupled with the module for switching the unit 14. The unit for generating disinfecting gas 14, may also consist of unit for generating negative or negative and positive ions. In the embodiment of Fig. 1, the apparatus has a display 5 and a handle 7 of the supply pipe 4. The handle 7 provides fixing of the supply pipe 4, when it is not in use. The pipe itself may be led outside the housing anywhere, in accordance with the specific technical requirements of a given situation. The display 5 may be used to indicate the time remaining until the end of the process, although a person skilled in the art will appreciate that the content and the form of the information displayed on the display 5 may vary. The upper part of the housing 2 preferably comprises a filter 6 for filtering the air sucked in by the vending machine. The filter 6 prevents dust and other undesirable elements from falling into the apparatus. In the illustrated embodiment, the filter 6 is positioned on the bottom side of the cover 12, i.e. on the bottom of a projecting portion of the cover, however a different position of the filter within the apparatus is also possible. Preferably, the filter 6 is shielded from above against precipitation. Fig. 2 shows a side view of the apparatus for gas disinfection 1 in the form of a vending machine. In the illustrated embodiment, the housing comprises side elements 11, a front element 13, a cover 12, a rear element 21 and a bottom part 19 illustrated in Fig. 3. Moreover, as shown in Fig. 2, the side wall of the housing includes a door 8 enabling access to the interior of the vending machine. The access door 8 has hinges 9 and a lock 10. However, in a simpler example of the housing 2, just one removable component such as the cover 12 or one of the side elements 11, would be enough to carry out repairs and maintenance. Fig. 3 shows in detail an exemplary inner structure of the apparatus for gas disinfection 1 according to the invention. In Fig. 3 the fare collection unit 3 is the unit for receiving coins comprising a tray 15. Fig. 3 shows an exemplary arrangement of the elements of the apparatus 1. For example, the elements of the apparatus are attached to a frame bearing structure 17. Preferably, the unit for generating disinfecting gas 14 is the unit for generating ozone. The unit for generating ozone is connected with the module 22 for starting the ozonation process, in this case the element constitutes a pressure switch used to switch on the unit. The unit for generating disinfecting gas is connected with a fan 13, e.g. a radial fan. The fan 13 sucks in the air and directs it into the unit for disinfecting gas generating 14. On the other side of the fan 13, the unit for generating disinfecting gas 14 is connected with the end of the connection pipe 16, the other end of which is led outside the vending machine. The supply pipe 4 is attached to the end of the connection pipe 16 led outside. The flexible supply pipe 4 allows the supply of ozone to a suitable place of the ozonated vehicle. The length, the cross-section and the diameter of the supply pipe 4 is adapted to the type of ozonated vehicles. The elements of the apparatus are connected to the connection unit 20. The interior of the housing preferably houses a heater 18 for heating the interior of the apparatus. Heating the interior of the apparatus prevents the elements of the apparatus from freezing and condensation of steam when using the apparatus in cold temperatures. The apparatus may also be equipped with an element which stops the process of disinfecting gas emissions, such as the "stop" button operated by the user, or a safety circuit breaker responding to specific adverse parameters of the apparatus. The apparatus may also comprise an element adjusting the amount of disinfecting gas dispensed.

The operation of the gas disinfection apparatus of the invention according to the embodiment of Fig. 1 will now be described. Upon a payment made by the user, the fan is started, which maintains the air flow through the unit for generating disinfecting gas, and the display shows the time remaining till the end of ozonation process. Once the unit for ozonation reaches an adequate air flow, the pressure switch switches on the unit for generating ozone. The generated stream of ozone is directed through the supply pipe to the vehicle, e.g. to a car. Having completed the ozonation process, the apparatus is automatically switched off. Moreover, disinfection of air conditioning components and ventilation ducts of a vehicle it is also possible by means of the present apparatus.

## Claims

1. Apparatus for gas disinfection (1), in particular for refreshing vehicles, comprising a housing (2) and an unit for generating disinfecting gas (14) positioned within the housing (2), and a fan (13) for sucking in the air, and for supplying the air to the unit for generating disinfecting gas (14), the fan being connected with the unit for generating disinfecting gas (14), the apparatus (1) further comprising a supply pipe (4) coupled with the unit for generating disinfecting gas (14), **characterized in that** the supply pipe (4) communicates with the outside of the apparatus (1) and the apparatus (1) is equipped with a fare collection unit (3) coupled with a module (22) for switching the unit for generating disinfecting gas (14).

2. Apparatus (1) according to claim 1, **characterized in that** the apparatus (1) further comprises an air filter (6) positioned within the plane of the housing.

3. Apparatus (1) according to claim 1, **characterized in that** the fare collection unit (3) is a unit for receiving coins or tokens.

4. Apparatus (1) according to claim 1, **characterized in that** the fare collection unit (3) is a unit for receiving notes.

5. Apparatus (1) according to claim 1, **characterized in that** the payment unit (3) is a unit for receiving payment cards.

6. Apparatus (1) according to claim 1, **characterized in that** the fare collection unit (3) is a unit for accepting payments by SMS.

7. Apparatus (1) according to claim 1, **characterized in that** the unit for generating disinfecting gas (14) is a unit for generating ozone.

8. Apparatus (1) according to claim 1, **characterized in that** the unit for generating disinfecting gas (14) is a unit for generating negative ions.

9. Apparatus (1) according to claim 1, **characterized in that** the unit for generating disinfecting gas (14) is a unit for generating negative and positive ions.

10. Apparatus (1) according to claim 1, **characterized in that** the apparatus is equipped with a display (5).

11. Apparatus (1) according to claims 1 or 9, **characterized in that** the module (22) for switching the unit for generating disinfecting gas is a pressure switch.

12. Apparatus (1) according to claims 1 or 9 or 10 or 11, **characterized in that** the apparatus (1) has a handle (7) for the supply pipe (4).

13. Apparatus (1) according to claims 1 or 9 or 10 or 11, **characterized in that** the housing (2) includes an access door (8).

14. Apparatus (1) according to claims 1 or 9 or 10 or 11 or 12, **characterized in that** the apparatus (1) has a heater (18) positioned within the housing (2).

15. Apparatus (1) according to claims 1 or 9 or 10 or 11 or 12 or 13, **characterized in that** the apparatus (1) has an element for adjusting the amount of the dispensed gas.

## Patentansprüche

1. Vorrichtung zur Gasdesinfektion (1), insbesondere zum Auffrischen von Fahrzeugen, umfassend ein Gehäuse (2) und eine Einheit zum Erzeugen des Desinfektionsgases (14), die im Gehäuse (2) angeordnet ist, ein Ventilator (13) zum Ansaugen von Luft, und zur Einspeisung der Luft in die Einheit zum Erzeugen des Desinfektionsgases (14), wobei der Ventilator mit der Einheit zum Erzeugen des Desinfektionsgases (14) verbunden ist, wobei die Vorrichtung (1) weiter ein Zuleitungsrohr (4) umfasst, das mit der Einheit zum Erzeugen des Desinfektionsgases (14) gekoppelt ist, **dadurch gekennzeichnet, dass** das Zuleitungsrohr (4) mit der Außenseite der Vorrichtung (1) kommuniziert und die Vorrichtung (1) mit einer Gebührenerhebungseinheit (3) ausgestattet ist, die mit einem Modul (22) zum Schalten der Einheit zum Erzeugen des Desinfektionsgases (14) gekoppelt ist.

2. Vorrichtung (1) nach Anspruch 1, **dadurch gekennzeichnet, dass** die Vorrichtung (1) weiter einen Luftfilter (6) umfasst, der in der Ebene des Gehäuses angeordnet ist.

3. Vorrichtung (1) nach Anspruch 1, **dadurch gekennzeichnet, dass** die Gebührenerhebungseinheit (3) eine Einheit zur Aufnahme von Münzen oder Wertmarken ist.

4. Vorrichtung (1) nach Anspruch 1, **dadurch gekennzeichnet, dass** die Gebührenerhebungseinheit (3) eine Einheit zur Aufnahme von Scheinen ist.

5. Vorrichtung (1) nach Anspruch 1, **dadurch gekennzeichnet, dass** die Zahlungseinheit (3) eine Einheit zur Aufnahme von Zahlungskarten ist.

6. Vorrichtung (1) nach Anspruch 1, **dadurch gekennzeichnet, dass** die Gebührenerhebungseinheit (3) eine Einheit zur Annahme von Zahlungen mittels SMS ist.

7. Vorrichtung (1) nach Anspruch 1, **dadurch gekennzeichnet, dass** die Einheit zum Erzeugen des Desinfektionsgases (14) eine Einheit zum Erzeugen von Ozon ist.

8. Vorrichtung (1) nach Anspruch 1, **dadurch gekennzeichnet, dass** die Einheit zum Erzeugen des Desinfektionsgases (14) eine Einheit zum Erzeugen von Negativionen ist.

9. Vorrichtung (1) nach Anspruch 1, **dadurch gekennzeichnet, dass** die Einheit zum Erzeugen des Desinfektionsgases (14) eine Einheit zum Erzeugen von Negativ- und Positivionen ist.

10. Vorrichtung (1) nach Anspruch 1, **dadurch gekennzeichnet, dass** die Vorrichtung mit einer Anzeige (5) ausgestattet ist.

11. Vorrichtung (1) nach Anspruch 1 oder 9, **dadurch gekennzeichnet, dass** das Modul (22) zum Schalten der Einheit zum Erzeugen des Desinfektionsgases ein Druckschalter ist.

12. Vorrichtung (1) nach Anspruch 1 oder 9 oder 10 oder 11, **dadurch gekennzeichnet, dass** die Vorrichtung (1) einen Griff (7) für das Zuleitungsrohr (4) aufweist.

13. Vorrichtung (1) nach Anspruch 1 oder 9 oder 10 oder 11, **dadurch gekennzeichnet, dass** das Gehäuse (2) eine Zugangstür (8) umfasst.

14. Vorrichtung (1) nach Anspruch 1 oder 9 oder 10 oder 11 oder 12, **dadurch gekennzeichnet, dass** die Vorrichtung (1) ein Heizgerät (18) aufweist, das im Gehäuse (2) angeordnet ist.

15. Vorrichtung (1) nach Anspruch 1 oder 9 oder 10 oder 11 oder 12 oder 13, **dadurch gekennzeichnet, dass** die Vorrichtung (1) ein Element zur Regelung der Menge des dosierten Gases aufweist.

## Revendications

1. Appareil (1) pour la désinfection de gaz, en particulier pour rafraîchir des véhicules, comprenant un boîtier (2) et une unité (14) pour la production de gaz de désinfection, positionnée à l'intérieur du boîtier (2), et un ventilateur (13) pour aspirer l'air, et pour fournir l'air à l'unité (14) pour la production de gaz de désinfection, le ventilateur étant relié à l'unité (14) pour la production de gaz de désinfection, l'appareil (1) comprenant en outre un tuyau d'alimentation (4) couplé à l'unité (14) pour la production de gaz de désinfection, **caractérisé en ce que** le tuyau d'alimentation (4) communique avec l'extérieur de l'appareil (1) et l'appareil (1) est équipé d'une unité (3) de perception des tarifs couplée à un module (22) pour mettre en marche l'unité (14) pour la production de gaz de désinfection.

2. Appareil (1) selon la revendication 1, **caractérisé en ce que** l'appareil (1) comprend en outre un filtre à air (6) positionné dans le plan du boîtier.

3. Appareil (1) selon la revendication 1, **caractérisé en ce que** l'unité (3) de perception des tarifs est une unité pour recevoir des pièces de monnaie ou des jetons.

4. Appareil (1) selon la revendication 1, **caractérisé en ce que** l'unité (3) de perception des tarifs est une unité pour recevoir des notes.

5. Appareil (1) selon la revendication 1, **caractérisé en ce que** l'unité de paiement (3) est une unité pour recevoir des cartes de paiement.

6. Appareil (1) selon la revendication 1, **caractérisé en ce que** l'unité (3) de perception des tarifs est une unité pour accepter des paiements par SMS.

7. Appareil (1) selon la revendication 1, **caractérisé en ce que** l'unité (14) pour la production de gaz de désinfection est une unité pour générer l'ozone.

8. Appareil (1) selon la revendication 1, **caractérisé en ce que** l'unité (14) pour la production de gaz de désinfection est une unité pour générer des ions négatifs.

9. Appareil (1) selon la revendication 1, **caractérisé en ce que** l'unité (14) pour la production de gaz de désinfection est une unité pour générer des ions négatifs et positifs.

10. Appareil (1) selon la revendication 1, **caractérisé en ce que** l'appareil est équipé d'un écran (5).

11. Appareil (1) selon les revendications 1 ou 9, **caractérisé en ce que** le module (22) pour mettre en marche l'unité pour la production de gaz de désinfection est un pressostat.

12. Appareil (1) selon les revendications 1 ou 9, ou 10, ou 11, **caractérisé en ce que** l'appareil (1) comporte une poignée (7) pour le tuyau d'alimentation (4).

13. Appareil (1) selon les revendications 1 ou 9, ou 10, ou 11, **caractérisé en ce que** le boîtier (2) comprend une porte d'accès (8).

14. Appareil (1) selon les revendications 1 ou 9, ou 10, ou 11, ou 12, **caractérisé en ce que** l'appareil (1) comporte un réchauffeur (18) positionné à l'intérieur du boîtier (2).

15. Appareil (1) selon les revendications 1 ou 9, ou 10, ou 11, ou 12, ou 13, **caractérisé en ce que** l'appareil (1) comporte un élément pour régler la quantité du gaz distribué.
